# DEMANDE DE BREVET EUROPEEN

(11) **EP 3 843 058 A1**
(43) Date de publication de la demande: **30.06.2021**
(21) Numéro de dépôt: 20216559.3
(22) Date de dépôt: 22.12.2020
(51) Int. Cl.: G08B 21/06, G08B 21/04

(54) **DISPOSITIF DE VEILLE POUR CONDUCTEUR DE VÉHICULE**

(30) Priorité: 23.12.2019 FR 1915456
(71) Demandeur: ALSTOM Transport Technologies, 93400 Saint-Ouen (FR)
(72) Inventeur: AYME, Nicolas, 17220 SAINTE SOULLE (FR)
(74) Mandataire: Lavoix

(57) **Abrégé**

L'invention concerne un dispositif de veille (10) pour conducteur de véhicule, comportant : un organe d'émission (12) d'un signal électrique ; et un dispositif d'alerte (16) apte à envoyer une alerte si ledit signal électrique n'est pas émis au cours d'un intervalle de temps prédéterminé. L'organe d'émission comporte un capteur piézoélectrique (20) apte à être relié à un membre (28) du conducteur, ledit capteur piézoélectrique étant apte à émettre le signal électrique sous l'effet d'une pression mécanique exercée sur ledit capteur.

## Description

La présente invention concerne un dispositif de veille pour conducteur de véhicule, du type comportant : un organe d'émission d'un signal électrique ; et un dispositif d'alerte apte à envoyer une alerte si ledit signal électrique n'est pas émis au cours d'un intervalle de temps prédéterminé.

Un tel dispositif de veille, décrit par exemple dans le document US2013/0135109, permet de vérifier en temps réel que le conducteur d'un véhicule, par exemple d'un véhicule ferroviaire, est dans un état de conscience lui permettant d'assurer la sécurité du véhicule.

Les systèmes de veille installés dans les véhicules ferroviaires actuels imposent par exemple au conducteur d'appuyer à intervalles réguliers sur un bouton ou sur une pédale installée dans la cabine de conduite du véhicule. De tels systèmes imposent des contraintes de position et de mouvement aux conducteurs.

La présente invention a pour but de proposer un dispositif de veille offrant une plus grande liberté de mouvement au conducteur, tout en garantissant la sécurité du véhicule.

A cet effet, l'invention a pour objet un dispositif de veille du type précité, dans lequel l'organe d'émission comporte un premier capteur piézoélectrique apte à être relié à un membre du conducteur, ledit premier capteur piézoélectrique étant apte à émettre le signal électrique sous l'effet d'une pression mécanique exercée sur ledit premier capteur.

Suivant d'autres aspects avantageux de l'invention, le dispositif de veille comporte l'une ou plusieurs des caractéristiques suivantes, prises isolément ou suivant toutes les combinaisons techniquement possibles :
- le dispositif de veille comprend en outre un support sur lequel est fixé le premier capteur piézoélectrique, ledit support étant configuré pour être associé à un membre du conducteur ;
- le support comporte un doigtier apte à être enfilé sur un doigt du conducteur ;
- l'organe d'émission comporte en outre un deuxième capteur piézoélectrique apte à être associé à un membre du conducteur, ledit deuxième capteur piézoélectrique étant apte à émettre le signal électrique sous l'effet d'une pression mécanique exercée sur ledit deuxième capteur ;
- le support comporte un gant apte à être enfilé sur une main du conducteur, les premier et deuxième capteurs piézoélectriques étant respectivement fixés à un premier et à un deuxième doigts dudit gant ;
- le dispositif de veille comprend en outre un module relié à l'organe d'émission et apte à communiquer au dispositif d'alerte une information relative au signal électrique émis par ledit organe d'émission ;
- le dispositif de veille comprend en outre un bracelet configuré pour être assemblé au membre du conducteur, le module étant logé dans ledit bracelet ;
- le dispositif de veille comprend en outre un détecteur de pulsation, apte à détecter un pouls du conducteur du véhicule, le dispositif de veille étant configuré pour communiquer au dispositif d'alerte une information relative audit pouls

L'invention se rapporte en outre à un procédé de mise en oeuvre d'un dispositif de veille tel que décrit ci-dessus, ledit procédé comprenant une étape telle que, si le signal électrique n'est pas émis par l'organe d'émission au cours d'un premier intervalle de temps prédéterminé, une première alerte est envoyée par le dispositif d'alerte.

Selon un mode de réalisation de l'invention, le procédé comprend ensuite une étape telle que, si le signal électrique n'est pas émis par l'organe d'émission au cours d'un deuxième intervalle de temps prédéterminé, une deuxième alerte est envoyée par le dispositif d'alerte.

L'invention sera mieux comprise à la lecture de la description qui va suivre, donnée uniquement à titre d'exemple non limitatif et faite en se référant aux dessins sur lesquels :
[Fig 1] [Fig 2] la figure 1 et la figure 2 sont des vues schématiques de dispositif de veille, respectivement selon un premier et un deuxième modes de réalisation de l'invention ; et

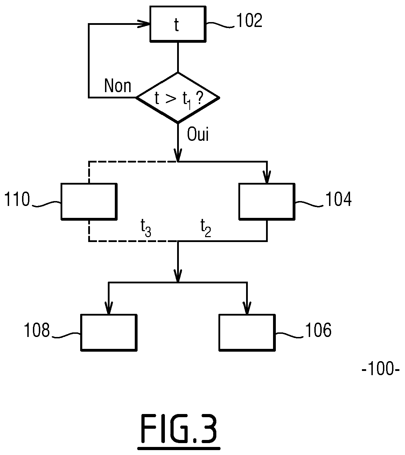

[Fig 3] la figure 3 est une représentation, sous forme de logigramme, d'un procédé de mise en oeuvre du dispositif de veille de la figure 1 ou de la figure 2.

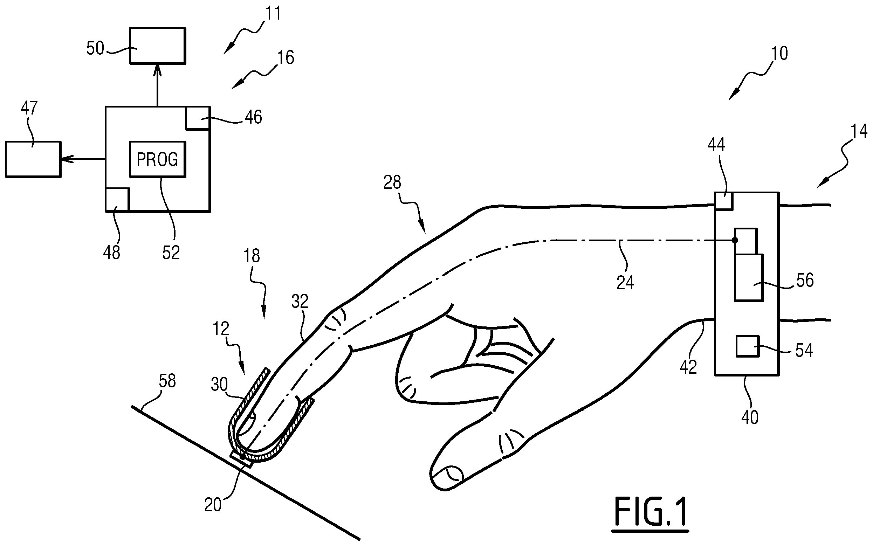

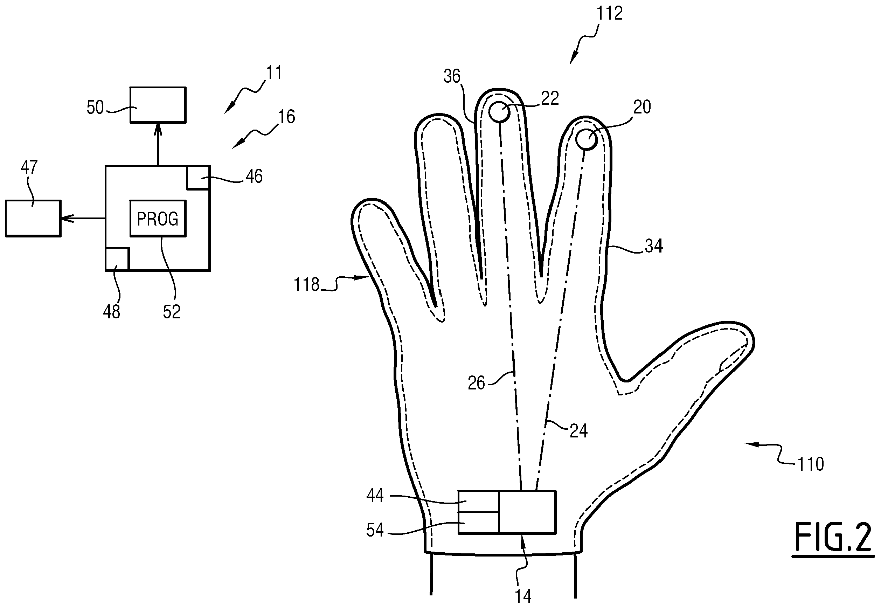

Les figures 1 et 2 représentent des dispositifs 10 et 110 de veille, respectivement selon un premier et selon un deuxième modes de réalisation de l'invention.

Les dispositifs 10 et 110 de veille sont destinés à équiper une cabine de conduite d'un véhicule 11, en particulier d'un véhicule ferroviaire de type tram. Les dispositifs 10 et 110 de veille sont notamment destinés à être utilisés par un conducteur dudit véhicule 11.

Les dispositifs 10 et 110 de veille seront décrits simultanément ci-après, les éléments communs étant désignés par les mêmes numéros de référence.

Le dispositif 10, 110 comporte: un organe 12, 112 d'émission d'un signal électrique ; un organe 14 de réception dudit signal électrique ; et un dispositif 16 d'alerte.

Dans les modes de réalisation représentés, le dispositif 10, 110 comporte en outre un support 18, 118.

L'organe d'émission 12, 112 comporte : au moins un capteur piézoélectrique 20, 22 ; et une liaison 24, 26 entre le ou chaque capteur piézoélectrique 20, 22 et l'organe de réception 14.

Le capteur piézoélectrique 20, 22 est apte à émettre le signal électrique sous l'effet d'une pression mécanique exercée sur ledit capteur.

L'au moins un capteur piézoélectrique 20, 22 est destiné à être relié à un membre, notamment à une main 28, du conducteur du véhicule 11 mentionné ci-dessus. A cet effet, le capteur piézoélectrique 20 ou chaque capteur piézoélectrique 20, 22 de l'organe d'émission 12, 112 est fixé au support 18, 118.

Dans le mode de réalisation de la figure 1, le support 18 comporte un doigtier 30 apte à être enfilé sur un doigt 32 du conducteur du véhicule 11. Le capteur piézoélectrique 20 est fixé à une extrémité dudit doigtier.

Dans le mode de réalisation de la figure 2, le support 118 est un gant apte à être enfilé sur la main 28 du conducteur. Chaque capteur piézoélectrique 20, 22 est fixé à une extrémité d'un doigt 34, 36 dudit gant.

L'organe de réception 14 est par exemple un module électronique. Dans le mode de réalisation de la figure 1, le support 18 comporte un bracelet 40 apte à être assemblé au poignet 42 du conducteur. Le module électronique 14 est logé dans ledit bracelet 40.

Dans le mode de réalisation de la figure 2, le module 14 est par exemple fixé au gant 118, au niveau du poignet.

La ou chaque liaison 24, 26 entre le module électronique 14 et le ou chaque capteur piézoélectrique 20, 22 est par exemple une liaison filaire.

Le module électronique 14 comporte un premier élément 44 de communication avec le dispositif d'alerte 16. Le premier élément 44 est par exemple un élément de communication sans fil, de type radio ou wi-fi.

Le dispositif d'alerte 16 est par exemple un boîtier électronique, de préférence installé dans la cabine de conduite du véhicule 11 équipé du dispositif 10, 110.

Le dispositif d'alerte 16 comporte un deuxième élément de communication 46, apte à coopérer avec le premier élément de communication 44 du module électronique 14. Le deuxième élément 46 est par exemple un élément de communication sans fil, de type radio ou wi-fi. En variante, les éléments 44 et 46 sont reliés par une liaison filaire.

Selon un premier mode de réalisation, le dispositif d'alerte 16 est apte à émettre un premier message d'alerte à l'intention du conducteur du véhicule 11. A cet effet, le dispositif d'alerte 16 est relié à un dispositif de signalisation 47 situé dans la cabine de conduite du véhicule 11. Le dispositif de signalisation 47 est par exemple un écran apte à afficher un message visuel, ou encore un haut-parleur apte à diffuser un message sonore.

Selon un deuxième mode de réalisation, éventuellement combinable au premier mode de réalisation précédent, le dispositif d'alerte 16 est apte à émettre un deuxième message d'alerte vers un poste de contrôle (non représenté) extérieur au véhicule 11. A cet effet, le dispositif d'alerte 16 comporte un élément 48 d'émission. L'élément 48 est par exemple un élément d'émission sans fil, de type radio.

Selon un mode de réalisation particulier, le dispositif d'alerte 16 est également relié à un système 50 de freinage d'urgence du véhicule 11.

Le dispositif d'alerte 16 mémorise un programme 52 permettant la mise en œuvre d'un procédé d'alerte associé au dispositif de veille 10. Les fonctionnalités du programme 52 seront décrites ci-après.

Selon un mode de réalisation de l'invention, le dispositif de veille 10, 110 comporte en outre un détecteur 54 de pulsation, relié au module électronique 14. Dans le mode de réalisation de la figure 1, le détecteur 54 est par exemple assemblé au bracelet 40. Dans le mode de réalisation de la figure 2, le détecteur 54 est par exemple assemblé au gant 118 au niveau du poignet.

Le détecteur 54 est notamment apte à détecter un pouls du conducteur du véhicule. Par ailleurs, le module électronique 14 est apte à communiquer au dispositif d'alerte 16 une information relative au pouls dudit conducteur.

Selon un mode de réalisation de l'invention, le dispositif de veille 10, 110 comporte en outre une source 56 d'énergie électrique, telle qu'une batterie, reliée au module électronique 14. Dans le mode de réalisation de la figure 1, la batterie 56 est par exemple logée dans le bracelet 40. Dans le mode de réalisation de la figure 2, la batterie est par exemple logée dans le gant 118 au niveau du poignet.

Un procédé de fonctionnement du dispositif 10 va maintenant être décrit.

Selon ledit procédé de fonctionnement, le conducteur du véhicule 11, dont la main 28 est reliée à l'organe d'émission 12, 112, active ledit organe d'émission. A cet effet, le conducteur exerce une pression mécanique sur le capteur piézoélectrique 20 de l'organe d'émission 12 ou sur l'un des capteurs piézoélectriques 20,22 de l'organe d'émission 12.

Afin d'exercer cette pression mécanique, le conducteur peut appuyer avec son doigt 32 sur une surface 58 de son choix, ladite surface se trouvant dans la cabine de conduite du véhicule 11, ou encore étant formée par un autre de ses doigts. Le conducteur a donc une grande liberté de mouvement pour l'activation de l'organe d'émission 12, 112.

Dans le mode de réalisation de la figure 2, le conducteur a le choix d'utiliser l'un ou l'autre des capteurs piézoélectriques 20,22 pour activer l'organe d'émission 12.

A chaque activation de l'organe d'émission 12, 112, un signal électrique est envoyé à l'organe de réception 14 par la liaison 24, 26. L'organe de réception 14 communique une information correspondante au dispositif d'alerte 16 au moyen des éléments 44, 46 de communication.

Le procédé de fonctionnement du dispositif 10 comprend par exemple un procédé d'alerte 100, mis en œuvre par le programme 52.

Dans une première étape 102, le dispositif d'alerte 16 contrôle une durée t écoulée depuis la dernière information reçue par le deuxième élément 46 de communication, concernant une activation de l'organe d'émission 12, 112.

Si la durée t est supérieure à un seuil t₁, le dispositif d'alerte 16 envoie un premier message d'alerte au dispositif de signalisation 47 (étape 104), afin d'alerter le conducteur. En cas d'oubli, ce dernier peut ainsi procéder à l'activation de l'organe d'émission 12, 112.

Puis, après un premier laps de temps t₂, si l'organe d'émission 12, 112 n'a toujours pas été activé, le dispositif d'alerte 16 envoie un deuxième message d'alerte au poste de contrôle (étape 106). En parallèle, de préférence, le dispositif d'alerte 16 active le système 50 de freinage d'urgence du véhicule 11 (étape 108). Ledit véhicule s'arrête alors automatiquement.

De manière optionnelle, en parallèle de l'émission du premier message d'alerte, le dispositif d'alerte 16 contrôle (étape 110) la pulsation détectée par le détecteur 54. Si aucune pulsation n'est détectée dans un deuxième laps de temps t₃, le dispositif d'alerte 16 envoie le deuxième message d'alerte au poste de contrôle (étape 106) et active éventuellement le système 50 de freinage d'urgence du véhicule 11 (étape 108).

De préférence, t₃ est choisi inférieur à t₂. Ainsi, en cas d'accident cardiaque du conducteur, la deuxième alerte peut être accélérée afin de secourir plus rapidement ledit conducteur.

Le dispositif de veille 10, 110 décrit ci-dessus permet une grande liberté de mouvement au conducteur du véhicule 11, ce qui améliore son confort et diminue sa fatigue. A plus long terme, une telle liberté de mouvement diminue également l'apparition de troubles musculo-squelettiques chez les conducteurs de véhicules.

## Revendications

1. Dispositif (10, 110) de veille pour conducteur de véhicule (11), comprenant : un organe d'émission (12, 112) d'un signal électrique ; et un dispositif d'alerte (16) apte à envoyer une alerte si ledit signal électrique n'est pas émis au cours d'un intervalle de temps (t₁) prédéterminé ; le dispositif de veille étant **caractérisé en ce que** l'organe d'émission comporte un premier capteur piézoélectrique (20, 22) apte à être relié à un membre (28) du conducteur, ledit premier capteur piézoélectrique étant apte à émettre le signal électrique sous l'effet d'une pression mécanique exercée sur ledit premier capteur.

2. Dispositif de veille selon la revendication 1, comprenant en outre un support (18, 118) sur lequel est fixé le premier capteur piézoélectrique, ledit support étant configuré pour être associé au membre du conducteur.

3. Dispositif de veille selon la revendication 2, dans lequel le support comporte un doigtier (30, 34, 36) apte à être enfilé sur un doigt (32) du conducteur.

4. Dispositif (110) de veille selon l'une quelconque des revendications précédentes, dans lequel l'organe d'émission (112) comporte en outre un deuxième capteur piézoélectrique (20, 22) apte à être associé à un membre du conducteur, ledit deuxième capteur piézoélectrique étant apte à émettre le signal électrique sous l'effet d'une pression mécanique exercée sur ledit deuxième capteur.

5. Dispositif de veille selon la revendication 4 prise en combinaison avec la revendication 2, dans lequel le support comporte un gant (118) apte à être enfilé sur une main du conducteur, les premier et deuxième capteurs piézoélectriques étant respectivement fixés à un premier (34) et à un deuxième (36) doigts dudit gant.

6. Dispositif de veille selon l'une quelconque des revendications précédentes, comprenant en outre un module (14) relié à l'organe d'émission (12, 112) et apte (44, 46) à communiquer au dispositif d'alerte (16) une information relative au signal électrique émis par ledit organe d'émission.

7. Dispositif (10) de veille selon la revendication 6, comportant en outre un bracelet (40) configuré pour être assemblé au membre du conducteur, le module (14) étant logé dans ledit bracelet.

8. Dispositif de veille selon l'une quelconque des revendications précédentes, comprenant en outre un détecteur (54) de pulsation, apte à détecter un pouls du conducteur du véhicule, le dispositif de veille étant configuré pour communiquer au dispositif d'alerte (16) une information relative audit pouls.

9. Procédé (100) de mise en œuvre d'un dispositif (10, 110) de veille selon l'une quelconque des revendications précédentes, comprenant une étape (104) telle que, si le signal électrique n'est pas émis par l'organe d'émission (12, 112) au cours d'un premier intervalle de temps (t₁) prédéterminé, une première alerte est envoyée par le dispositif d'alerte (16).

10. Procédé (100) selon la revendication 9, comprenant ensuite une étape (106) telle que, si le signal électrique n'est pas émis par l'organe d'émission (12, 112) au cours d'un deuxième intervalle de temps (t₂) prédéterminé, une deuxième alerte est envoyée par le dispositif d'alerte (16).
